# EUROPEAN PATENT APPLICATION

(11) **EP 3 719 144 A1**
(43) Date of publication of application: **07.10.2020**
(21) Application number: 19382252.5
(22) Date of filing: 05.04.2019
(51) Int. Cl.: C12Q 1/6876, G16B 25/10

(54) **MIR-151A-3P AS AN UNIVERSAL ENDOGENOUS CONTROL FOR EXOSOME CARGO NORMALIZATION**

(71) Applicant: Fundación para la Investigación Biomédica del Hospital Universitario de la Paz (FIBHULP), 28046 Madrid (ES)
(72) Inventor: IBÁÑEZ DE CÁCERES, Inmaculada, 28046 Madrid (ES); DE CASTRO CARPEÑO, Javier, 28046 Madrid (ES); JIMÉNEZ HERNÁNDEZ, Julia, 28046 Madrid (ES); RODRÍGUEZ ANTOLÍN, Carlos, 28046 Madrid (ES); RODRÍGUEZ JIMÉNEZ, Carmen, 28046 Madrid (ES); ROSAS ALONSO, Rocío, 28046 Madrid (ES); CRUZ CASTELLANOS, Patricia, 28046 Madrid (ES); BURDIEL HERENCIA, Miranda, 28046 Madrid (ES); PERNÍA ARIAS, Olga, 28046 Madrid (ES); DIESTRO TEJEDA, María Dolores, 28046 Madrid (ES); ESTEBAN RODRÍGUEZ, Mª Isabel, 28046 Madrid (ES)
(74) Representative: Hoffmann Eitle

(57) **Abstract**

The present invention responds to the need to provide an endogenous miRNA for quantifying the amount of one or more targets miRNA in exosomes using normalization, that that does not vary within the different tumor types and within the different chemotherapeutic treatments. This is essential to be able to perform robust analysis of the expression of exosomal miRNAs in these pathologies. So far, other types of endogenous controls have been used that are not strictly exosomal, thus reducing their reliability as normalizers. Therefore, for the first time, an endogenous control specific for the exosomal compartment would be available.

## Description

### Technical field of the invention

The invention relates to methods for quantification of the amount of target miRNAs in exosomes using normalization. The invention further relates to kits for quantifying the amount of a target miRNA in exosomes using a reference value.

### Background of the invention

The use of exosomal miRNAs as cancer biomarkers has aroused the interest of many researchers around the world since Valadi et al. reported for the first time the presence of miRNAs in exosomes in 2007 (Valadi, H. et al. Exosome-mediated transfer of mRNAs and microRNAs is a novel mechanism of genetic exchange between cells. Nat. Cell Biol. 9, 654-659 (2007). MicroRNAs (miRNAs) are short non-coding RNAs of 18-25 nucleotides that regulate gene expression at the post-transcriptional level. These miRNAs bind messenger RNAs (mRNA) by complementarity of sequence inhibiting their translation and promoting their degradation. Moreover, each mRNA can be regulated by different miRNAs, which makes them especially interesting since they have functionality in themselves being able to produce changes in the expression of many genes. More specifically, their great potential as biomarkers inside exosomes lies in their easy accessibility and non-invasiveness, their greater stability and integrity with respect to circulating miRNAs as well as their enrichment in cancer patients' blood.

Exosomes are nanosized vesicles (30-140 nm) that most cell types secrete as a mechanism of cellular communication. It has been described that their content is not arbitrary and there is a cargo selection depending on the cell type and the context. For example, Zhang et al. postulated that rat cardiomyoblasts release exosomes enriched in cardioprotective miRNAs under hypoxia conditions (Zhang, J. et al. Overexpression of Exosomal Cardioprotective miRNAs MitigatesHypoxia-lnduced H9c2 Cells Apoptosis. Int. J. Mol. Sci. 18, 2017). Moreover, it has been reported that breast milk exosomes carry immune-related miRNAs that could be essential for the infant immune system development (Zhou, Q. et al. Immune-related microRNAs are abundant in breast milk exosomes. Int. J. Biol. Sci. 8, 118-123, 2012).

However, although in this last decade literature related with exosomal miRNAs has increased exponentially, there is still a lack of a strong endogenous control that can be used to normalize miRNA levels in these small vesicles. In this sense, establishing a reference is essential to reliably compare different experimental conditions or pathological status. In fact, the use of an inadequate endogenous control can bias the analysis of a study resulting in uncertain conclusions.

Thus far, the most frequent endogenous controls used to normalize exosomal miRNAs are miR-16 and small nuclear RNA (snRNA) U6 (or RNU6B) in cancer (Tanaka, Y. et al. Clinical impact of serum exosomal microRNA-21 as a clinical biomarker in human esophageal squamous cell carcinoma. Cancer 119, 1159-1167, 2013). Tokuhisa, M. et al. Exosomal miRNAs from Peritoneum Lavage Fluid as Potential Prognostic Biomarkers of Peritoneal Metastasis in Gastric Cancer. PLOS ONE 10 , e0130472, 2015) (Li, X. J., Ren, Z. J., Tang, J. H. & Yu, Q. Exosomal MicroRNA MiR-1246 Promotes Cell Proliferation, Invasion and Drug Resistance by Targeting CCNG2 in Breast Cancer. Cell. Physiol. Biochem. Int. J. Exp. Cell. Physiol. Biochem. Pharmacol. 44 , 1741-1748, 2017)(Que, R., Ding, G., Chen, J. & Cao, L. Analysis of serum exosomal microRNAs and clinicopathologic features of patients with pancreatic adenocarcinoma. World J. Surg. Oncol.11 , 219, 2013)(Warnecke-Eberz, U., Chon, S.-H., Hölscher, A. H., Drebber, U. &Bollschweiler, E. Exosomal onco-miRs from serum of patients with adenocarcinoma of the esophagus:comparison of miRNA profiles of exosomes and matching tumor. Tumour Biol. J. Int. Soc.Oncodevelopmental Biol. Med. 36, 4643-4653, 2015)(Samsonov, R. et al. Lectin-induced agglutination method of urinary exosomes isolation followed by mi-RNA analysis: Application for prostate cancer diagnostic. The Prostate 76, 68-79, 2016)(Wang, J. et al. Combined detection of serum exosomal miR-21 and HOTAIR as diagnostic and prognostic biomarkers for laryngeal squamous cell carcinoma. Med. Oncol. NorthwoodLond. Engl. 31, 148, 2014)(Matsumura, T. et al. Exosomal microRNA in serum is a novel biomarker of recurrence in human colorectal cancer. Br. J. Cancer 113, 275-281, 2015), as well as in other pathologies often without validating their stability in exosomes. The use of miR-16 is usually justified with studies made in plasma (Kanaoka, R. et al. Usefulness of Plasma Exosomal MicroRNA-451a as a Noninvasive Biomarker for Early Prediction of Recurrence and Prognosis of Non-Small Cell Lung Cancer.Oncology 94 , 311-323, 2018) (Xie, Z. et al. Salivary microRNAs as promising biomarkers for detection of esophageal cancer. PloS One 8, e57502, 2013), and the use of RNU6B normally is not even referenced, probably assuming that its stability in the whole cell can be extended to exosomes. However, exosome biogenesis takes place in a specific compartment inside the cell and as their content is selected during the formation, it is necessary to find a concrete endogenous control for these vesicles. Recently, some studies have demonstrated the unsuitability of these non-coding RNAs as endogenous controls in exosomes (Gouin, K. et al. A comprehensive method for identification of suitable reference genes in extracellular vesicles. J. Extracell. Vesicles 6, 1347019, 2017)(Li, Y. et al. Identification of Endogenous Controls for Analyzing Serum Exosomal miRNA in Patients with Hepatitis B or Hepatocellular Carcinoma. Dis. Markers 2015, 2015)(Ni, Q. et al. Different signatures of miR-16, miR-30b and miR-93 in exosomes from breast cancer and DCIS patients. Sci. Rep. 8 , 12974, 2018).

In the present invention, we identify miR-151a as a potential endogenous control for exosome miRNA content in exosomes by using a high-throughput approach combining NGS (small_RNA -seq) from the exosomes isolated from various tumor cell lines and from plasma of NSCLC and ovarian cancer patients. miR-151a shall allow measuring exosomal miRNA levels in a more reliable and accurate way, enabling researchers and the industry to find strong biomarkers in cancer exosomes.

### Brief description of the figures

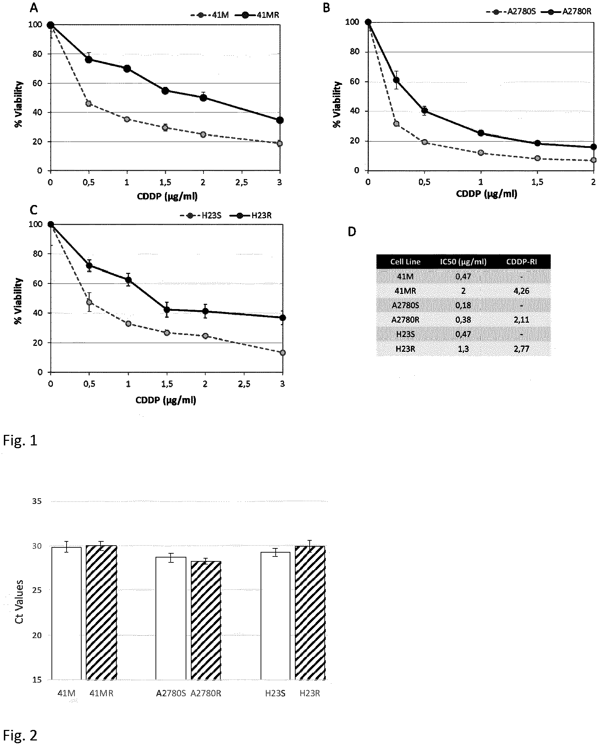

**Figure 1**. Sensitive and resistant lines used in the present invention. 41M / MR (A), A2780S / R (B), H23S / R (C). (D) IC50 of each of the lines for the drug cisplatin and resistance index (CDDP-RI), calculated as (IC50 Resistant) / (IC50 Sensitive).
**Figure 2**. Ct values of miR-151a in sensitive and resistant lines. The error bars correspond to the standard deviation value.

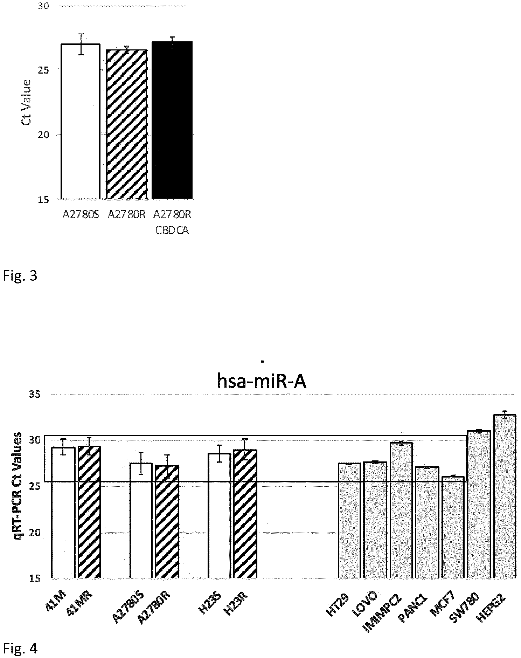

**Figure 3****.** Ct values of miR-151a in sensitive line A2780 (S), resistant to CDDP (R) and resistant to Carboplatin (CBDCA) (R CBDCA). The error bars correspond to the standard deviation value.
**Figure 4**. Amplification by qRT-PCR of the exosomal miRNA hsa-miR-151a in cell lines of 7 tumor types. Ovarian cancer (41M, 41MR, A2780S, A2780R), lung (H23S, H23R), colorectal (HT29, LOVO), pancreas (IMIMPC2, PANC1), breast (MCF7), bladder (Sw780) and liver (HEPG2). As observed, miR-151a is present in all the lines analyzed and maintains minimal intra- and inter-tumoral variation.

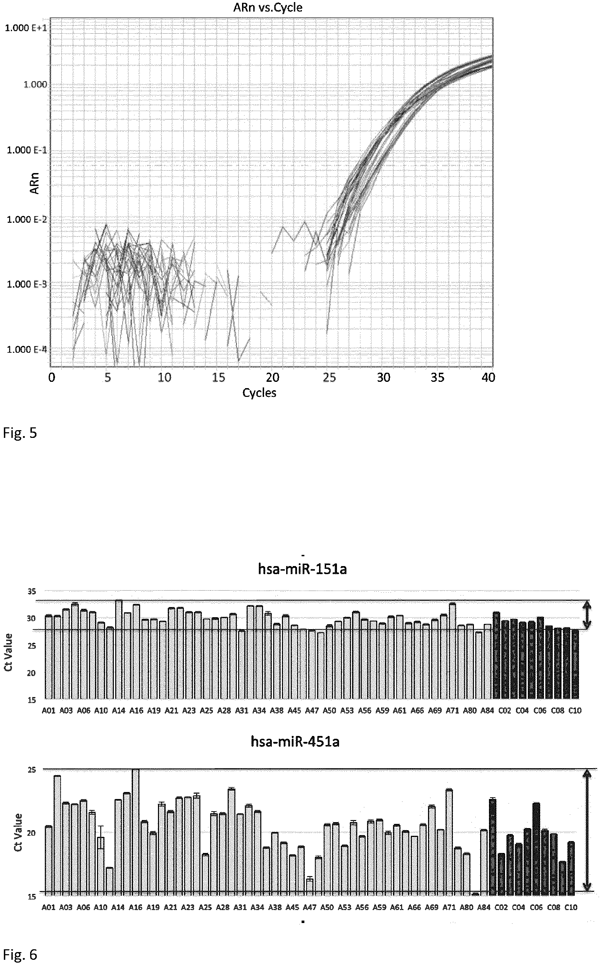

**Figure 5**. Example of a qRT-PCR amplification of the exosomal miRNAs hsa-miR-151a in a total of 10 samples: 4 from tumor cell lines of different tissue origin and 6 from lung cancer patients. In this example we observed a homogeneous amplification in all the samples analyzed, irrespective of the type of sample or tissue analyzed, which supports the robustness of this miRNA as an endogenous control of exosomal miRNAs.
**Figure 6****.** Amplification by qRT-PCR of the exosomal miRNAs hsa-miR-151a and hsa-451a in plasma samples of 51 lung cancer patients (grey bars) and 10 healthy donors (black bars). The arrows show the variability in amplification cycles between the samples analyzed. As we can see in the figure the high and homogeneous values of miR-151a in plasma samples from healthy donors and patients with lung cancer in advanced stages, provide solid data of the use of that miRNA as an endogenous control. On the contrary, in the case of the other miRNA analyzed, hsa-miR-451a, said homogeneity is not observed.

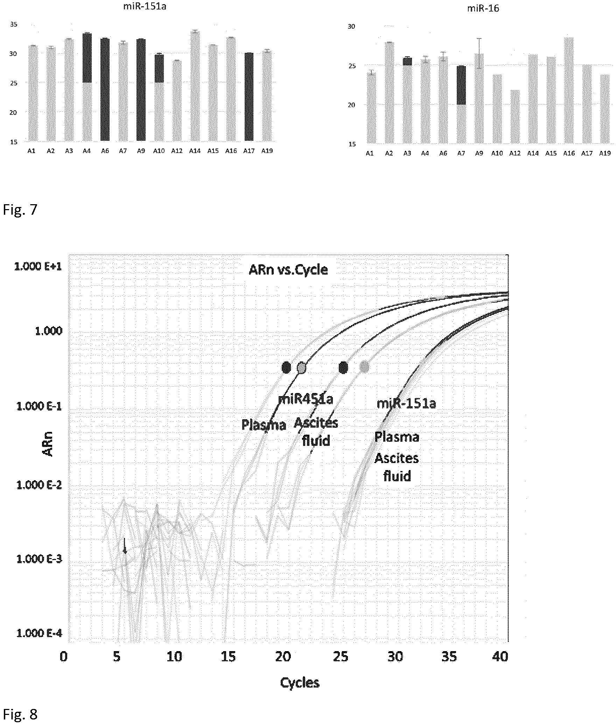

**Figura 7.** Amplification by qRT-PCR of the exosomal miRNAs hsa-miR-151a and the gold standard miR-16 in the same plasma samples (PL) of 14 lung cancer patients. We can observe a strong less variability in the miR151a levels in all samples in comparison with the observed with miR-16.
**Figure 8****.** Amplification by qRT-PCR of the exosomal miRNAs hsa-miR-151a and hsa-miR-451a in paired plasma and ascites samples from two ovarian cancer patients (light grey: patient 1, dark grey: patient 2). As we can see, the levels of miRNA hsa-miR-151a remain homogeneous in all four samples.

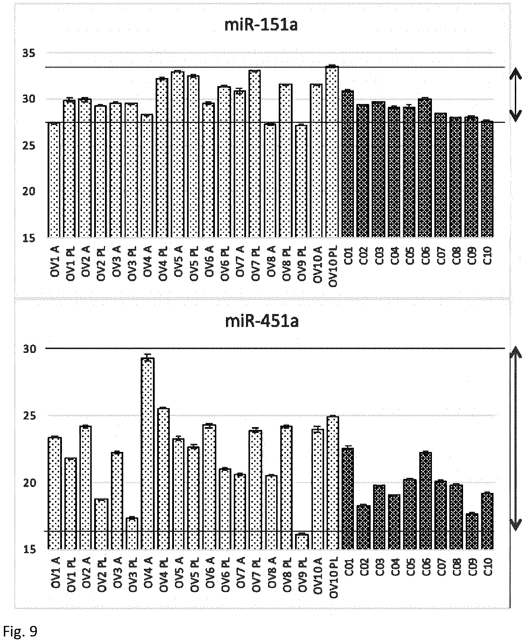

**Figure 9****.** Amplification by qRT-PCR of the exosomal miRNAs hsa-miR-151a and miR-451-a in plasma (PL) and paired ascites (A) samples of 10 ovarian cancer patients (grey bars) and 10 healthy donors (black bars). The arrows show the variability in amplification cycles between the samples analyzed. As we can see in the figure the high and homogeneous values of miR-151a in plasma and ascites samples from healthy donors and patients with ovarian cancer, provide solid data of the use of that miRNA as an endogenous control. On the contrary, in the case of the other miRNA analyzed, hsa-miR-451a, said homogeneity is not observed.

### Description of the invention

### Definitions

As used herein, the term "microRNA" (miRNA) includes human miRNAs, mature single stranded miRNAs, precursor miRNAs, and variants thereof, which may be naturally occurring or synthetic. Synthetic or naturally occurring miRNAs may be modified to include chemical groups other than hydroxy or phosphate at their 5' termini, sugar, and/or base modifications. In some instances the term "miRNA" also includes primary miRNA transcripts and duplex miRNAs. The term includes target miRNAs, miRNAs, and reference miRNAs (see below). The term "mature," when modifying miRNA or a specific miRNA, refers to the mature sequence(s) processed from the corresponding pre-miRNA sequence that are present in a biological sample. The sequences for particular miRNAs, including human mature and precursor sequences, are reported in the miRBase: Sequences Database (http:/microrna.sanger.ac.uk; Griffiths-Jones et al., Nucleic Acids Research, 2006, 34, Database Issue, D140-D144; Griffiths-Jones, Nucleic Acids Research, 2004, 32, Database Issue, D109-D111). The skilled artisan will appreciate that scientific consensus regarding the precise nucleic acid sequence for a given miRNA, in particular for mature forms of the miRNAs, may change with time. MiRNAs detected by assays of this application include naturally occurring sequences for the miRNAs.
miR-151a-3p is identified in table 1 with the accession number.

### Detailed description of the invention

The present invention responds to the need to provide an endogenous miRNA for quantifying the amount of one or more targets miRNA in exosomes using normalization, wherein the content/level/presence of the endogenous miRNA does not vary within the different tumor types and within the different chemotherapeutic treatments or among patients or healthy controls. This is essential to be able to perform a robust analysis of the expression of exosomal miRNAs in these pathologies. So far, other types of endogenous controls have been used that are not strictly exosomal, thus reducing their reliability as normalizers. Therefore, for the first time, an endogenous control specific for the exosomal compartment would be available.

Therefore, herein, the inventors propose a novel, more standardized method for the normalization of PCR or NGS, preferably RT-qPCR, experiments in the exosomal compartment. The invention is based on the surprising finding that miR-151a-3p is stably represented in the exosomal compartment of healthy individuals which are not related to a disease and in the exosomal compartment of patients suffering from ovarian, lung, colorrectal, pancreas, breast, bladder and liver cancer. This miRNA may therefore be used as a universal endogenous control for miRNA experiments on circulating exosomes and tissue exosomes miRNAs.

The invention thus provides a method for quantifying the exosomal microRNAs cargo or content of any given target microRNA/s (miRNA) in a biological sample isolated from a human subject, wherein said biological sample preferably comprises circulating exosomes or tissue exosomes miRNAs, and said method comprises the following steps:
a. Measuring the exosomal microRNAs content or level of one or more target miRNAs in the biological sample;
b. Measuring the exosomal microRNAs content or level of at least reference miRNA miR-151a-3p, in the biological sample; and
c. normalizing the one or more target miRNAs measurements based on the amount of at least said reference miRNA.

Preferably, said method further comprises amplifying the target miRNA and the reference miRNA in the reaction volume. Said amplification preferably includes real time polymerase chain reaction amplification. An advantage of miR-151a-3p is that it has shown to be very stable in the exosomes from biological samples obtained from plasma, serum, whole blood and tissue.

In a preferred embodiment of the method of the invention said biological sample containing the exosomes is obtained from serum, plasma, whole blood, tissue (i.e biopsies) or platelets.

In addition, the present invention further provides an *in vitro* use of miR-151a-3p as an endogenous control for exosome cargo normalization in a biological sample, more particularly in a biological sample containing circulating exosomes or tissue exosomes miRNAs. Preferably, said biological sample is selected from the list consisting of whole blood, serum, plasma, the secretome, or a tissue sample. Preferably, said biological sample is a tissue sample from ovaries, lung, colorrectal tissue, pancreas, breast, bladder or liver.

The invention further provides a kit suitable for quantifying the amount of any given target microRNA (miRNA) in the exosomes from any biological sample, a (human) cell culture, or the secretome obtained from a human cell, containing exosomes, preferably circulating exosomes or tissue exosomes miRNAs, that uses or comprises miR-151a-3p as an endogenous control for the normalization of said target miRNA in said exosomes.

The invention further provides a kit for quantifying the amount of a target miRNA in the exosomes of a biological sample, more particularly in a biological sample containing circulating exosomes or tissue exosomes miRNAs, comprising an amplification primer set, comprising at least one primer sequence that is complementary to a portion of reference miRNA miR-151a-3p, and a second primer sequence that is complementary to a portion of a target miRNA. That is to say, the kit comprises a first primer comprising a sequence that is complementary to a portion of the target miRNA and a second primer comprising a sequence that is complementary to a portion of the reference miRNA, wherein the first and second primers are preferably distinguishably labelled by using i.e two different probes.

The invention still further provides a kit as defined in any of the above aspects for exosome cargo normalization. Preferably, for use in a method of quantifying the amount of any given target microRNA (miRNA) in the exosomes from a biological sample isolated from a human subject, more particularly in a biological sample comprising circulating exosomes or tissue exosomes miRNAs, the method comprising: measuring the amount of the target miRNA in the exosomes from the biological sample; determining the amount of at least the reference miRNA miR-151a-3p in the exosomes from the said biological sample; and normalizing the target miRNA measurement based on the amount of said reference miRNA, wherein, preferably, said first reference miRNA and said target miRNA are not the same.

As already indicated, it is noted that the above method or kit for quantifying or normalizing the exosomal microRNAs cargo or content of any given target microRNA/s (miRNA) can be performed, apart from in human biological samples, in cell cultures or in the secretomes, as long as said cell cultures or secretome comprise exosomes; wherein preferably said cell cultures or secretomes are of human origin.

### I. Methods to Determine the Amount of a miRNA

Many methods of quantifying miRNAs are contemplated. Any reliable, sensitive, and specific method for quantifying miRNAs within exosomes can be used in the present invention. In some embodiments provided, a target miRNA or the reference miRNA is preferably amplified prior to or during quantification. In other embodiments, the miRNA is not amplified as part of the quantification process.

### A. Amplification Reactions

Many methods exist for amplifying miRNA nucleic acid sequences such as mature miRNAs, precursor miRNAs, and primary miRNAs. Suitable nucleic acid polymerization and amplification techniques include reverse transcription (RT), polymerase chain reaction (PCR), real-time PCR (quantitative PCR (q-PCR)), nucleic acid sequence-base amplification (NASBA), ligase chain reaction, multiplex ligatable probe amplification, invader technology (Third Wave), rolling circle amplification, in vitro transcription (IVT), strand displacement amplification, transcription-mediated amplification (TMA), RNA (Eberwine) amplification, and other methods that are known to persons skilled in the art. In certain preferred embodiments, more than one amplification method is used, such as reverse transcription followed by real time PCR (Chen et al., Nucleic Acids Research, 33(20):el79, 2005).

A typical PCR reaction includes multiple amplification steps, or cycles that selectively amplify target nucleic acid species. A typical PCR reaction includes three steps: a denaturing step in which a target nucleic acid is denatured; an annealing step in which a set of PCR primers (forward and reverse primers) anneal to complementary DNA strands; and an elongation step in which a thermostable DNA polymerase elongates the primers. By repeating these steps multiple times, a DNA fragment is amplified to produce an amplicon, corresponding to the target DNA sequence. Typical PCR reactions include 20 or more cycles of denaturation, annealing, and elongation. In many cases, the annealing and elongation steps can be performed concurrently, in which case the cycle contains only two steps. Since mature miRNAs are single-stranded, a reverse transcription reaction (which produces a complementary cDNA sequence) is performed prior to PCR reactions. Reverse transcription reactions include the use of, e.g., a RNA -based DNA polymerase (reverse transcriptase) and a primer.

In PCR and q-PCR methods, for example, a set of primers is used for each target sequence. In certain embodiments, the lengths of the primers depend on many factors, including, but not limited to, the desired hybridization temperature between the primers, the target nucleic acid sequence, and the complexity of the different target nucleic acid sequences to be amplified. In preferred embodiments, a primer is about 15 to about 35 nucleotides in length. In other preferred embodiments, a primer is equal to or fewer than 15, 20, 25, 30, or 35 nucleotides in length. In additional preferred embodiments, a primer is at least 35 nucleotides in length.

In preferred embodiments of the invention, forward primers can comprise at least one sequence that anneals to a target miRNA and/or to the reference miRNA and alternatively can comprise an additional 5' non-complementary region. In another embodiment, reverse primers can be designed to anneal to the complement of a reverse transcribed miRNA. The reverse primer may be independent of the target miRNA or reference miRNA sequence, and multiple target miRNAs and the reference miRNAs may be amplified using the same reverse primer. Alternatively, a reverse primer may be specific for a target miRNA and for the reference miRNA.

In some preferred embodiments, two or more miRNAs are amplified in a single reaction volume (one or more target miRNAs and the reference miRNA, for example). Normalization may alternatively be performed in separate reaction volumes. One preferred embodiment includes multiplex q-PCR, such as qRT-PCR, which enables simultaneous amplification and quantification of at least one miRNA of interest and at least the reference miRNA (miR-151a-3p) in one reaction volume by using more than one pair of primers and/or more than one probe. The primer pairs may comprise at least one amplification primer that uniquely binds each miRNA, and the probes are preferably labelled such that they are distinguishable from one another, thus allowing simultaneous quantification of multiple miRNAs. Multiplex qRT-PCR has research and diagnostic uses, including but not limited to detection of miRNAs for diagnostic, prognostic, and therapeutic applications.

A single combined reaction for q-PCR, is desirable for several reasons: (1) decreased risk of experimenter error, (2) reduction in assay-to-assay variability, (3) decreased risk of target or product contamination, and (4) increased assay speed. The qRT-PCR reaction may further be combined with the reverse transcription reaction by including both a reverse transcriptase and a DNA -based thermostable DNA polymerase. When two polymerases are used, a "hot start" approach may be used to maximize assay performance (U.S. Pat. Nos. 5,411,876 and 5,985,619). For example, the components for a reverse transcriptase reaction and a PCR reaction may be sequestered using one or more thermoactivation methods or chemical alteration to improve polymerization efficiency (U.S. Pat. Nos. 5,550,044, 5,413,924, and 6,403,341).

### B. Detection of miRNAs

In preferred embodiments, labels, dyes, or labelled probes and/or primers are used to detect amplified or unamplified miRNAs. Depending on the sensitivity of the detection method and the abundance of the target, for example, amplification may or may not be required prior to detection. One skilled in the art will recognize the detection methods where miRNA amplification is preferred.

A probe or primer may include Watson-Crick bases or modified bases. Modified bases include, but are not limited to, the AEGIS bases (from Eragen Biosciences), which have been described, e.g., in U.S. Pat. Nos. 5,432,272, 5,965,364, and 6,001,983. In certain preferred embodiments, bases are joined by a natural phosphodiester bond or a different chemical linkage. Different chemical linkages include, but are not limited to, a peptide bond or a Locked Nucleic Acid (LNA) linkage, which is described, e.g., in U.S. Pat. No. 7,060,809.

In a preferred embodiment, oligonucleotide probes or primers present in a multiplex amplification are suitable for monitoring the amount of amplification product produced as a function of time. In certain preferred embodiments, probes having different single stranded versus double stranded character are used to detect the nucleic acid. Probes include, but are not limited to, the 5'-exonuclease assay (e.g., TaqMan(TM)) probes (see U.S. Pat. No. 5,538,848), stem-loop molecular beacons (see, e.g., U.S. Pat.Nos. 6,103,476 and 5,925,517), stemless or linear beacons (see, e.g., WO 9921881, U.S. Pat.Nos. 6,485,901 and 6,649,349), peptide nucleic acid (PNA) Molecular Beacons (see, e.g., U.S. Pat.Nos. 6,355,421 and 6,593,091), linear PNA beacons (see, e.g. U.S. Pat.No. 6,329,144), non-FRET probes (see, e.g., U.S. Pat. No. 6,150,097), Sunrise(TM)/AmplifluorB(TM)probes (see, e.g., U.S. Pat. No. 6,548,250), stem-loop and duplex Scorpion(TM) probes (see, e.g., U.S. Pat. No. 6,589,743), bulge loop probes (see, e.g., U.S. Pat. No. 6,590,091), pseudo knot probes (see, e.g., U.S. Pat. No. 6,548,250), cyclicons (see, e.g., U.S. Pat. No. 6,383,752), MGB Eclipse(TM) probe (Epoch Biosciences), hairpin probes (see, e.g., U.S. Pat. No. 6,596,490), PNA light-up probes, antiprimer quench probes (Li et al., Clin. Chem. 53:624-633 (2006)), self-assembled nanoparticle probes, and ferrocene-modified probes described, for example, in U.S. Pat. No. 6,485,901.

In certain preferred embodiments, one or more of the primers in an amplification reaction can include a label. In yet further embodiments, different probes or primers comprise detectable labels that are distinguishable from one another. In some preferred embodiments a nucleic acid, such as the probe or primer, may be labelled with two or more distinguishable labels.

In preferred embodiments, a label is attached to one or more probes and has one or more of the following properties: (i) provides a detectable signal; (ii) interacts with a second label to modify the detectable signal provided by the second label, e.g., FRET (Fluorescent Resonance Energy Transfer); (iii) stabilizes hybridization, e.g., duplex formation; and (iv) provides a member of a binding complex or affinity set, e.g., affinity, antibody-antigen, ionic complexes, hapten-ligand (e.g., biotin-avidin). In still other embodiments, use of labels can be accomplished using any one of a large number of known techniques employing known labels, linkages, linking groups, reagents, reaction conditions, and analysis and purification methods.

miRNAs can be detected by direct or indirect methods. In a direct detection method, one or more miRNAs are detected in the exosomes by a detectable label that is linked to a nucleic acid molecule. In such methods, the miRNAs may be labelled prior to binding to the probe. Therefore, binding is detected by screening for the labelled miRNA that is bound to the probe. The probe is optionally linked to a bead in the reaction volume.

In certain preferred embodiments, nucleic acids are detected in the exosomes by direct binding with a labelled probe, and the probe is subsequently detected. In one preferred embodiment of the invention, the nucleic acids, such as amplified miRNAs, are detected using FlexMAP Microspheres (Luminex) conjugated with probes to capture the desired nucleic acids. Some methods may involve detection with polynucleotide probes modified with fluorescent labels or branched DNA (bDNA) detection, for example.

In other preferred embodiments, nucleic acids are detected in the exosomes by indirect detection methods. In such an embodiment, it is preferred that a biotinylated probe is combined with a streptavidin-conjugated dye to detect the bound nucleic acid. The streptavidin molecule binds a biotin label on amplified miRNA, and the bound miRNA is detected by detecting the dye molecule attached to the streptavidin molecule. In one embodiment, the streptavidin-conjugated dye molecule comprises Phycolink(R) Streptavidin R-Phycoerythrin (PROzyme). Other conjugated dye molecules are known to persons skilled in the art.

Labels include, but are not limited to: light-emitting, light-scattering, and light-absorbing compounds which generate or quench a detectable fluorescent, chemiluminescent, or bioluminescent signal (see, e.g., Kricka, L., Nonisotopic DNA Probe Techniquies, Academic Press, San Diego (1992) and Garman A., Non-Radioactive Labeling, Academic Press (1997).). Fluorescent reporter dyes useful as labels include, but are not limited to, fluoresceins (see, e.g., U.S. Pat. Nos. 5,188,934, 6,008,379, and 6,020,481), rhodamines (see, e.g., U.S. Pat.Nos. 5,366,860, 5,847,162, 5,936,087, 6,051,719, and 6,191,278), benzophenoxazines (see, e.g., U.S. Pat.No. 6,140,500), energy-transfer fluorescent dyes, comprising pairs of donors and acceptors (see, e.g., U.S. Pat. Nos. 5,863,727; 5,800,996; and 5,945,526), and cyanines (see, e.g., WO 9745539), lissamine, phycoerythrin, Cy2, Cy3, Cy3.5, Cy5, Cy5.5, Cy7, FluorX (Amersham), Alexa 350, Alexa 430, AMCA, BODIPY 630/650, BODIPY 650/665, BODIPY-FL, BODIPY-R6G, BODIPY -TMR, BODIPY-TRX, Cascade Blue, Cy3, Cy5, 6-FAM, Fluorescein Isothiocyanate, HEX, 6-JOE, Oregon Green 488, Oregon Green 500, Oregon Green 514, Pacific Blue, REG, Rhodamine Green, Rhodamine Red, Renographin, ROX, SYPRO, TAMRA, Tetramethylrhodamine, and/or Texas Red, as well as any other fluorescent moiety capable of generating a detectable signal. Examples of fluorescein dyes include, but are not limited to, 6-carboxyfluorescein; 2',4',1,4,-tetrachlorofluorescein; and 2',4',5',7',1,4-hexachlorofluorescein. In certain preferred embodiments, the fluorescent label is selected from SYBR-Green, 6-carboxyfluorescein ("FAM"), TET, ROX, VIC(TM), and JOE. For example, in certain preferred embodiments, labels are different fluorophores capable of emitting light at different, spectrally-resolvable wavelengths (e.g., 4-differently colored fluorophores); certain such labeled probes are known in the art and described above, and in U.S. Pat. No. 6,140,054. A dual labeled fluorescent probe that includes a reporter fluorophore and a quencher fluorophore is used in some preferred embodiments. It will be appreciated that pairs of fluorophores are chosen that have distinct emission spectra so that they can be easily distinguished.

In still a further preferred embodiment, labels are hybridization-stabilizing moieties which serve to enhance, stabilize, or influence hybridization of duplexes, e.g., intercalators and intercalating dyes (including, but not limited to, ethidium bromide and SYBR-Green), minor-groove binders, and cross-linking functional groups (see, e.g., Blackburn et al., eds. "DNA and RNA Structure" in Nucleic Acids in Chemistry and Biology (1996)).

In further preferred embodiments, methods relying on hybridization and/or ligation to quantify miRNAs may be used, including oligonucleotide ligation (OLA) methods and methods that allow a distinguishable probe that hybridizes to the target nucleic acid sequence to be separated from an unbound probe. As an example, HARP-like probes, as disclosed in U.S. Publication No. 2006/0078894 (incorporated herein by reference) may be used to measure the quantity of miRNAs. In such methods, after hybridization between a probe and the targeted nucleic acid, the probe is modified to distinguish the hybridized probe from the unhybridized probe. Thereafter, the probe may be amplified and/or detected. In general, a probe inactivation region comprises a subset of nucleotides within the target hybridization region of the probe. To reduce or prevent amplification or detection of a HARP probe that is not hybridized to its target nucleic acid, and thus allow detection of the target nucleic acid, a post-hybridization probe inactivation step is carried out using an agent which is able to distinguish between a HARP probe that is hybridized to its targeted nucleic acid sequence and the corresponding unhybridized HARP probe. The agent is able to inactivate or modify unhybridized HARP probe such that it cannot be amplified.

In an additional preferred embodiment of the method, a probe ligation reaction may be used to quantify miRNAs. In a Multiplex Ligation-dependent Probe Amplification (MLPA) technique(Schouten et al., Nucleic Acids Research 30:e57 (2002)) pairs of probes which hybridize immediately adjacent to each other on the target nucleic acid are ligated to each other only in the presence of the target nucleic acid. In some preferred embodiments, MLPA probes have flanking PCR primer binding sites. MLPA probes can only be amplified if they have been ligated, thus allowing for detection and quantification of target miRNA or reference miRNA.

### II. Normalization

Methods of normalization and kits for exosome cargo normalization are provided herein. The methods correct for exosome sample-to-exosome sample variability by comparing a target measurement in a sample to the reference miRNA control presented herein. Normalization of miRNA quantification assays reduces systematic (non-biological) and non-systematic differences between samples, and is critical for accurate measurement of differential miRNA content, for example.

The accurate measurement of the biological differential content between two groups of exosomes samples (or samples comprising exosomes or exosome content) is the goal of many miRNA qRT-PCR assays. Yet, miRNA levels in qRT-PCR reactions can vary from one exosomes samples (or samples comprising exosomes or exosome content) to the next for reasons that may be technical or biological.

Technical reasons may include variabilities in tissue procurement or storage, inconsistencies in RNA extraction or quantification, or differences in the efficiency of the reverse transcription and/or PCR steps. Biological reasons may include exosomes sample (or samples comprising exosomes or exosome content)-to-sample heterogeneity in cellular populations, differences in bulk transcriptional activity, or alterations in specific miRNA content that is linked to an aberrant biological program (e.g., a disease state). Given the multiplicity of sources that can contribute to differences in miRNA quantification, results from qRT-PCR assays should be normalized against a relevant endogenous target or targets to minimize controllable variation, and permit definitive interpretations of nominal differences in exosome cargo normalization.

Preferred embodiments comprise multiplex methods for quantifying and normalizing the amount of a target miRNA in a biological sample. In a preferred embodiment of the invention, the amount of one or more target miRNAs is measured in the exosomes in a reaction volume, and the amount of at least the reference miRNA of the present invention measured in the reaction volume. The amount of target miRNA is normalized based on the amount of the reference miRNA.

For experiments using only the reference miRNA of the present invention for normalization, the data are normalized to the measured quantity of said one reference miRNA. When two or more reference miRNAs are used as normalizers, a mean of the normalizers (e.g. arithmetic mean or geometric mean) is preferably used, depending on the nature of the quantification data. For example, the threshold cycle (Ct) values obtained from q-PCR experiments may be normalized to the geometric mean of two or more normalizers. Data represented on a linear scale (absolute expression data) may be normalized to an arithmetic mean of normalizers. Additional methods of combining normalizers are also contemplated, such as weighted averages.

In some embodiments, expression levels may be normalized using a comparative Ct method for relative quantification between samples or sample types. The general methods for conducting such assays are described, e.g., in Real-Time PCR Systems: Applied Biosystems 7900HT Fast Real-Time PCR System, and 7300/7500 Real-Time PCR Systems, Chemistry Guide, Applied Biosystems, 2005, Part No. 4348358.

Many additional methods of normalization are well known to those skilled in the art, and all normalization methods are contemplated. Those skilled in the art will recognize the appropriate normalization methods for each quantification and detection method described herein.

### III. Reference MiRNA miR-151a-3p

Preferred embodiments of the invention include measuring the amount of at least miR-151a-3p **reference** miRNA in the exosomes contained in a biological sample, more particularly in a biological sample containing circulating exosomes or tissue exosomes miRNAs, or contained in a cell culture or in the secretome obtained or isolated from human cells, and normalizing the amount of a target miRNA to the amount of said reference miRNA. In this sense and as shown in the example and figures, miR-151a-3p is shown herein to be stably represented in the exosomes from any human origin, and does not show significant differential content in the exosomes from healthy or diseased individuals. Therefore, in the normalization methods provided herein, the amount of target miRNA in the exosomes of any biological sample of human origin can be normalized to the amount of at least miR-151a-3p as a reference miRNA in the exosomes from the biological sample.

A "biological sample" is any sample or specimen derived from a human that contains exosomes. For example, the biological sample may be a patient sample. A "patient sample" is any biological specimen from a patient. The term includes, but is not limited to, biological fluids such as blood, serum, plasma, urine, cerebrospinal fluid, tears, saliva, lymph, dialysis fluid, lavage fluid, semen, and other liquid samples, as well as cells and tissues of biological origin. The term also includes cells isolated from a human or cells derived therefrom, including cells in culture, cell supernatants, and cell lysates. It further includes organ or tissue culture-derived fluids, tissue biopsy samples, tumor biopsy samples, stool samples, and fluids extracted from physiological tissues, as well as cells dissociated from solid tissues, tissue sections, and cell lysates. A biological sample may be obtained or derived from tissue types including but not limited to lung, liver, placenta, bladder, brain, heart, colon, thymus, ovary, adipose, stomach, prostate, uterus, skin, muscle, cartilage, breast, spleen, pancreas, kidney, eye, bone, intestine, esophagus, lymph nodes and glands. The term "biological sample" encompasses samples that have been manipulated in any way after their procurement, such as by treatment with preservatives, cellular disruption agents (e.g. lysing agents), solubilization, purification, or enrichment for certain components, such as polynucleotides, in certain aspects. Also, derivatives and fractions of patient samples are included. A sample may be obtained or derived from a patient having, suspected of having, or recovering from a disease or pathological condition. Diseases and pathological conditions include, but are not limited to, proliferative, inflammatory, immune, metabolic, infectious, and ischemic diseases. Diseases (e.g. cancers) also include neural, immune system, muscular, reproductive, gastrointestinal, pulmonary, cardiovascular, and renal diseases, disorders, and conditions. In a preferred embodiment said biological sample comprises serum, whole blood or platelets

### IV. Kits

In another aspect, the invention provides kits of reagents and macromolecules for carrying out the normalization assays provided herein. In one embodiment, the invention provides a kit for quantifying a target miRNA sequence and the reference miRNA sequence (miR-151a-3p) in a reaction volume. The kits include nucleic acid sequences that are identical or complementary to a portion of at least one target miRNA and at least to the reference miRNA as defined above, for the detection of the target miRNA and the reference miRNA. In one embodiment, the kits comprise at least one primer for the detection of a reference miRNA and a target miRNA. In another embodiment, the kits comprise at least one probe specific to a reference miRNA and a target miRNA. The sequence-specific primers are preferably distinguishably labelled, allowing detection of at least the reference miRNA and at least one target miRNA in a single reaction volume.

The kits further optionally comprise an enzyme for carrying out the method described herein, including but not limited to a polymerase such as a reverse transcriptase or a DNA polymerase, or a ligase. In certain embodiments, the kits preferably include nucleic acid molecules that are identical or complementary to a target miRNA and/or a reference miRNA. Such molecules may serve as absolute standards for creating standard curves to quantify the unknown levels of target in the sample of interest.

In various embodiments, the kits preferably comprise multiple amplification primer sets, wherein at least one of the primers in each of the primer sets comprises a sequence that is complementary to a portion of at least two miRNAs, such as a target miRNA and the reference miRNA. In other embodiments, the kits preferably further comprise at least two probes complementary to a portion of at least two miRNAs. The kit preferably also comprises reagents for reverse transcribing RNA to a DNA template and/or reagents, including primers, for amplification of the target DNA. Such a kit preferably includes one or more buffers, such as a reaction, amplification, and/or a transcription buffer, compounds for preparing a RNA sample, for preparing a DNA sample, and components for isolating and/or detecting an amplification product, such as a probe or label, for example.

In some embodiments, kits of the invention preferably include one or more of the following (consistent with methods, reagents, and compositions discussed above): components for sample purification, including a lysis buffer with a chaotropic agent; a glass-fiber filter or column; an elution buffer; a wash buffer; an alcohol solution; and a nuclease inhibitor. The components of the kits may be packaged either in aqueous media or in lyophilized form, for example, and will be provided in a suitable container. The components of the kit may be provided as dried powder(s). When reagents and/or components are provided as a dry powder, the powder can be reconstituted by the addition of a suitable solvent. It is envisioned that the solvent may also be provided in another container. The container will generally include at least one vial, test tube, flask, bottle, syringe, and/or other container means, into which the solvent is placed, optionally aliquoted. The kits may also comprise a second container means for containing a sterile, pharmaceutically acceptable buffer and/or other solvent.

Where there is more than one component in the kit, the kit also will generally contain a second, third, or other additional container into which the additional components may be separately placed. However, various combinations of components may be comprised in a container. The kits of the present invention will also typically include a means for containing the RNA, and any other reagent containers in close confinement for commercial sale. Such containers may include injection or blow-molded plastic containers into which the desired vials are retained. When the components of the kit are provided in one and/or more liquid solutions, the liquid solution is an aqueous solution, with a sterile aqueous solution being particularly preferred.

Such kits may also include components that preserve or maintain DNA or RNA, such as reagents that protect against nucleic acid degradation. Such components may be nuclease or RNase-free or protect against RNases, for example. Any of the compositions or reagents described herein may be components in a kit.

The above disclosure generally describes the present invention. Unless defined otherwise, all technical and scientific terms used herein have the same meaning as those commonly understood by one of ordinary skill in the art to which this invention belongs. A more complete understanding can be obtained by reference to the following specific example which is provided herein for purposes of illustration only, and is not intended to limit the scope of the invention.

### Example

### Material and methods

### - Cell culture

Eleven human cancer cell lines H23, H1299, H727, HT29, LoVo, IMIMPC2, PANC1, MCF7, SW780, A2780 and HEPG2 were purchased from the ATCC (Manassas, VA) or ECACC (Sigma-Aldrich, Spain). All of them were maintained in RPMI or DMEM supplemented with 10% exosome-depleted FBS, according with manufacturer specification. FBS was depleted of bovine exosomes by ultracentrifugation at 100,000 × g for 16h at 4ºC. The CDDP-resistant variants H23R and A2780R were established by exposing cells to increasing doses of cisplatin. The CDDP sensitive and resistant ovarian cancer cell lines 41M and 41MR were provided by Dr.Kelland (UK) and maintained in DMEM supplemented with 10% exosome-depleted FBS.

### - NSCLC and Ovarian Cancer samples and data collection

Plasma samples from 51 advanced NSCLC patients (stages IIIA to IV) and nine plasma and ascites paired samples from ovarian cancer patients were collected in La Paz University Hospital before received any platinum-based treatment. We also collected 10 plasma samples from healthy donors. Follow-up was conducted according to the criteria of the medical oncology division of La Paz University Hospital. All the samples were processed following the standard operating procedures with the appropriate approval of the Human Research Ethics Committees, including informed consent within the context of research. Clinical, pathological and therapeutic data were recorded by an independent observer and blinded for statistical analysis.

### - Exosome and RNA isolation

Cell line-derived exosomes, used for small RNA-seq and proteomic analysis, were isolated by miRCURY Exosome Isolation Kit (Exiqon, Denmark) according to manufacturer's instructions. Circulating exosomes and their miRNA content from plasma and ascites samples were obtained with exoRNeasy Serum/Plasma Midi Kit (Qiagen, Germany).

### -Small RNA-seq

Small RNA seq from 41M, 41MR, A2780S, A2780R, H23S and H23R cell lines was developed by ArrayStar-miRNAseq-LM160802. Code: IlbanezldiPaz. Concentration values were measured with NanodropandBioanalyzerranking between 17,3 and 96,3ng/ul. Concentrations were normalized and massively sequenced. Obtained Reads were 5-10M. Data were normalized in order to find those miRNAs with less variability between samples. miR151-3p was the one with the highest p. value, showing the least difference in expression between samples analyzed.

| Identifier | RNA (ng/ul) |
|---|---|
| 41M | 96.3 |
| 41MR | 53.4 |
| A2780S | 36.1 |
| A2780R | 45.7 |
| H23S | 30.3 |
| H23R | 17.3 |

### - qRT-PCR

RNA from cell line exosomes and from plasma samples was extracted as described above. Total miRNAs were retrotranscribed using TaqMan™ Advanced miRNA cDNA Synthesis Kit (Thermofisher Scientific). Quantitative RT-PCR analysis was performed using TaqMan™ Advanced miRNA assays (Thermofisher Scientific) and TaqMan™ Universal PCR Master Mix (Applied Biosystems, Spain) or TaqMan™ Fast Advanced Master Mix, depending on the assay (Table 1). Samples were analysed in triplicate using the HT7900 Real-Time PCR System (Applied Biosystems, USA).

### - Results

### Comprehensive searching of an endogenous exosomal miRNA

To find a suitable endogenous control specific for miRNA content of tumor-derived exosomes, we performed small RNA-seq on total RNA extracted from exosomes isolated from the culture media of three pairs of lung and ovarian cancer cell lines sensitive and resistant to cisplatin (CDDP) H23S/H23R, A2780S/A2780R and 41S/41R. We wanted to compare the miRNA profile of the six cell lines in order to identify candidates that were stable between two different tumor types (lung and ovarian), between two different lines of the same tissue origin (A2780 and 41) and also in each line compared with the resistant to CDDP subtype. This last comparison represents an external aggression to the cell, which is previously reported to change gene expression and methylation profile. We aimed to identify an invariant miRNA inside the exosomes and through different conditions and sample origins. For this purpose, we ordered the miRNAs from highest to lowest expressed in all the cell lines, with at least one count per million readings in all the experimental conditions or samples, and with the lowest difference in the absolute values (log 2 FC RvsS) between the resistant and sensitive phenotype for each of the cell lines. Obtaining a list of 9 miRNAs that potentially could be used as endogenous control in tumor-derived exosomes. Additionally, we checked the expression of the candidates in 30 cancer types from The Cancer Genome Atlas (TCGA) in order to increase the strength of the candidates chosen (Table 1).

**Table 1**

| **miRNA Name** | **Accesion Number** | **Length (bp)** | **Assay ID** | **Mean Internal Variability (\|log₂FC RvsS\|)** | **Internal Variability in ovarian cancer (\|log₂FC RvsS\|)** | **Internal Variability in lung cancer (\|log₂FC RvsS\|)** | **Log₂(Mean Expression) Variance in 30 cancer types (TCGA)** |
|---|---|---|---|---|---|---|---|
| hsa-miR-151a-3p | M10000809 | 21 | 477919_mir | 0,084 | 0,105 | 0,041 | 0,32 |
| hsa-miR-22-5p | M10000078 | 22 | 4779878_mir | 0,169 | 0,066 | 0,376 | 0,58 |
| hsa-miR-502-3p | M10003186 | 22 | | 0,193 | 0,263 | 0,052 | 0,61 |
| hsa-miR-221-3p | M10000298 | 23 | 477981_mir | 0,257 | 0,356 | 0,054 | 1,67 |
| hsa-miR-1183 | M10006276 | 27 | | 0,276 | 0,350 | 0,129 | 0 |
| hsa-miR-27a-3p | M10000085 | 21 | 478384_mir | 0,278 | 0,296 | 0,242 | 6,67 |
| hsa-let-7l-5p | M10000434 | 22 | | 0,297 | 0,441 | 0,011 | 0,60 |
| hsa-miR-411-5p | M10003675 | 21 | | 0,323 | 0,196 | 0,579 | 3,05 |
| hsa-miR-196b-5p | M10001150 | 22 | | 0,329 | 0,218 | 0,550 | 3,67 |

To validate the expression levels of candidates with an alternative methodology, we performed qRT-PCR using Taqman probes. We found no significant changes in the expression of miR-151a (see figures 1 to 6). Moreover, as shown in figure 7, amplification by qRT-PCR of the exosomal miRNAs hsa-miR-151a and **the gold standard miR-16** in the same plasma samples (PL) of 14 lung cancer patients. We can observe a strong less variability in the miR151a levels in all samples in comparison with the observed with 16s. In addition, the high and homogeneous values of miR-151a in plasma and ascites samples from healthy donors and patients with ovarian cancer shown in figure 9 provide solid proof of the use of said miRNA as an endogenous control.

## Claims

1. *In vitro* use of miR-151a-3p as an endogenous control for the normalization of one or more target miRNAs present in the exosomes of a biological sample isolated from a human subject, in human cell cultures or in the secretome obtained from human cells.

2. The use according to claim 1, wherein said biological sample is selected from the list consisting of whole blood, serum, plasma or a tissue sample.

3. The use according to any of claims 1 or 2, wherein said biological sample is a tissue sample from ovaries, lung, colorectal tissue, pancreas, breast, bladder or liver.

4. An *in vitro* method of quantifying the exosomal microRNAs cargo or content of a given target microRNA (miRNA) in a biological sample isolated from a human subject, in human cell cultures or in the secretome obtained from human cells, wherein said method comprises the following steps:
a. Measuring the exosomal microRNAs content of one or more target miRNAs in the biological sample, cell culture or secretome;
b. Measuring the exosomal microRNAs content or level of at least reference miRNA miR-151a-3p, in the said biological sample, cell culture or secretome; and,
c. normalizing the one or more target miRNAs measurements based on the amount of at least said reference miRNA.

5. The method according to claim 4, wherein said biological sample is selected from the list consisting of whole blood, serum, plasma or a tissue sample.

6. The method according to any of claims 4 to 5, wherein said biological sample is a tissue sample from ovaries, lung, colorrectal tissue, pancreas, breast, bladder or liver.

7. The method according to any one of claims 4 to 6, further comprising amplifying the target miRNA and the at least one reference miRNA in a reaction volume.

8. The method according to any one of claims 4 to 7, wherein the amplification includes real-time polymerase chain reaction amplification.

9. A kit suitable for measuring or quantifying the amount of any given target microRNA (miRNA) in the exosomes from any biological sample, cell culture or secretome isolated from human cells, more particularly in a biological sample containing circulating exosomes or tissue exosomes miRNAs, that uses miR-151a-3p as an endogenous control for the normalization of the content of said target miRNA in said exosomes.

10. A kit for quantifying the amount of one or more target miRNAs in the exosomes of a biological sample isolated from a human subject, or in a cell culture or secretome isolated from human cells, more particularly in a biological sample containing circulating exosomes or tissue exosomes miRNAs, comprising an amplification primer set comprising at least one primer sequence that is complementary to a portion of reference miRNA miR-151a-3p, and a second primer sequence that is complementary to a portion of said target miRNA.

11. In vitro use of the kit as defined in any of claims 9 or 10, for exosome cargo normalization.

12. In vitro use of the kit as defined in any of claims 9 or 10, in a method of quantifying the amount of any given target microRNA (miRNA) in the exosomes from a biological sample isolated from a human subject, more particularly in a biological sample comprising circulating exosomes or tissue exosomes miRNAs, the method comprising: measuring the amount of the target miRNA in the exosomes from the biological sample; determining the amount of at least the reference miRNA miR-151a-3p in the exosomes from the said biological sample; and normalizing the target miRNA measurement based on the amount of said reference miRNA, wherein, preferably, said first reference miRNA and said target miRNA are not the same.
